(19) Europäisches Patentamt
European Patent Office
Office européen des brevets

(11) Publication number: **0 060 119**
**B1**

(12) # EUROPEAN PATENT SPECIFICATION

(45) Date of publication of patent specification: **10.04.85**

(21) Application number: **82301165.5**

(22) Date of filing: **08.03.82**

(51) Int. Cl.$^4$: **C 07 D 205/08**, C 07 D 409/12, C 07 D 403/04, A 61 K 31/395, A 61 K 31/40

(54) **Azetidinone compounds and their preparation.**

(30) Priority: **09.03.81 US 241989**

(43) Date of publication of application: **15.09.82 Bulletin 82/37**

(45) Publication of the grant of the patent: **10.04.85 Bulletin 85/15**

(84) Designated Contracting States: **BE CH DE FR GB IT LI LU NL SE**

(56) References cited:
**THE JOURNAL OF ORGANIC CHEMISTRY, vol. 46, no. 17, 14th August 1981, pages 3568-70, (USA); W.A. SPITZER et al.: "Azetidinone antibiotics. 22. A rearrangement of oxoazetidinesulfinic acids to haloazetidinones"**

(73) Proprietor: **ELI LILLY AND COMPANY**
**307, East McCarty Street**
**Indianapolis Indiana 46285 (US)**

(72) Inventor: **Goodson, Theodore, Jr.**
**4045 Devon Drive**
**Indianapolis Indiana 46226 (US)**
Inventor: **Spitzer, Wayne Alfred**
**5501 Moller Road**
**Indianapolis Indiana 46254 (US)**

(74) Representative: **Hudson, Christopher Mark et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 060 119 B1

**Description**

This invention relates to 4-fluoroazetidinone compounds and to a process for the preparation thereof from penicillins.

4-Chloroazetidinones are used as intermediates in the process described by S. Wolfe, U.S. Patent Nos. 3,948,927, 3,950,352, 4,013,653, and 4,071,512, and Canadian Journal of Chemistry, 52, 3990—3999 (1974) and *53* 497—512 (1975) in the preparation of ß-lactam antibiotics.

It has now been discovered that a 4-fluoro-azetidinone of formula 1

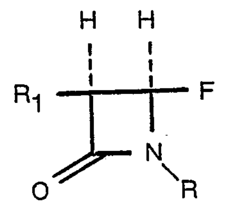

(1)

wherein $R_1$ is amino or

1) an imido group of the formula

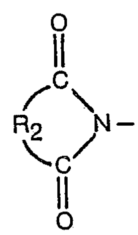

wherein $R_2$ is $C_2$—$C_4$ alkylene or 1,2-phenylene;

2) an amido group of the formula

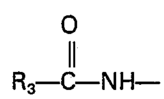

wherein $R_3$ is

a) hydrogen, $C_1$—$C_4$ alkyl, halomethyl, cyanomethyl, benzyloxy, p-nitrobenzyloxy, t-butyloxy, 2,2,2-trichloroethoxy, or p-methoxybenzyloxy;

b) the group R′, wherein R′ in phenyl or phenyl substituted by 1 or 2 halogens, hydroxy, protected hydroxy, nitro, cyano, $C_1$—$C_4$ alkyl, or $C_1$—$C_4$ alkoxy;

c) a group of the formula

$$R''—(Q)_m—CH_2—$$

wherein R″ is R′ as defined above, 1,4-cyclohexadienyl, thienyl or furyl; m is 0 or 1; and Q is O or S; with the limitation that when m is 1, R″ is R′;

d) a group of the formula

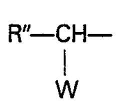

wherein R″ is as defined above, and W is hydroxy, protected hydroxy, carboxy, protected carboxy, amino, or protected amino;

3) an imidazolidinyl group of the formula

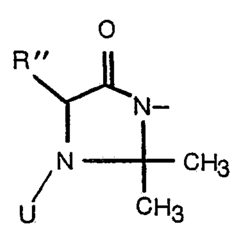

wherein R″ is as defined above and U is nitroso or acetyl; or $R_1$ is

4) an imido group of the formula

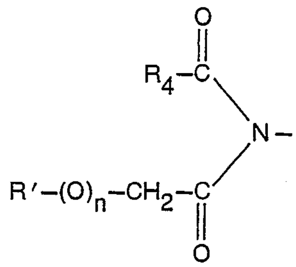

wherein R′ is as defined above, n is 0 or 1, and $R_4$ is $C_1$—$C_4$ haloalkyl, $C_1$—$C_4$ alkoxy, or 2,2,2-trichloroethoxy; R is a carboxy-substituted or esterified carboxy-substituted butenyl group represented by the formulas

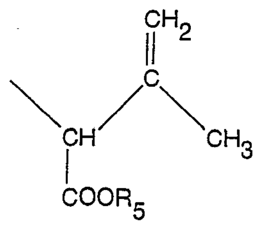
,
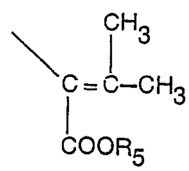

wherein $R_5$ is hydrogen or a carboxy protecting group; or a pharmaceutically-acceptable salt thereof is useful for the preparation of a β-lactam antibiotic.

A 4-fluoroazetidinone compound of the above formula wherein $R_1$ is other than amino, benzyloxycarbonylamino, p-nitrobenzyloxycarbonylamino, t-butyloxycarbonylamino, 2,2,2-trichloroethoxycarbonylamino, or p-methoxybenzyloxycarbonylamino, and R is the carboxy-substituted butenyl group having the double bond, α,β is also an antibacterial agent.

In the above definition the term "$C_1$—$C_4$ alkyl" refers to methyl, ethyl, n-propyl, iso-propyl, n-butyl, sec-butyl, and t-butyl; "$C_1$—$C_4$ alkoxy" refers to methoxy, ethoxy, n-propoxy, iso-propoxy, n-butoxy, and t-butoxy; "halogen" refers to fluoro, chloro, or bromo; while the terms "protected hydroxy", "protected amino" and "protected carboxy" refer to the hydroxy, amino, and carboxy groups substituted with a conventional blocking group used for the temporary protection of the hydroxy, amino or carboxy group.

Illustrative of the particular acylamino group,

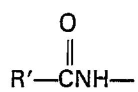

are benzamido, 2,6-dimethoxybenzamido, 4-chlorobenzamido, 4-methylbenzamido, 3,4-dichlorobenzamido, 4-cyanobenzamido, 3-bromobenzamido, 3-nitrobenzamido and the like.

Exemplary of the acylamino group

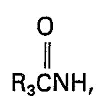

when $R_3$ is a group of the formula R″—$(Q)_m CH_2$— and m is 0, are cyclohexa-1,4-dienylacetamido, phenylacetamido, 4-chlorophenylacetamido, 3-methoxyphenylacetamido, 3-cyanophenylacetamido, 3-methylphenylacetamido, 4-bromophenylacetamido, 4-ethoxyphenylacetamido, 4-nitrophenylacetamido, 3,4-dimethoxyphenylacetamido 2-thienylacetamido, 3-thienylacetamido and the like; and when m is 1 and Q is 0, representative acylamino groups are phenoxyacetamido, 4-cyanophenoxyacetamido, 4-chlorophenoxyacetamido, 3,4-dichlorophenoxyacetamido, 2-chlorophenoxyacetamido, 4-methoxyphenoxyacetamido, 2-ethoxyphenoxyacetamido, 3,4-dimethylphenoxyacetamido, 4-isopropylphenoxyacetamido, 3-cyanophenoxyacetamido, 3-nitrophenoxyacetamido and like substituted phenoxyacetamido groups; and when m is 1 and Q is S, representative groups are phenylthioacetamido, 2,5-dichlorophenylthioacetamido, 4-bromophenylthioacetamido, 4-methoxyphenylthioacetamido, 4-tolylthioacetamido, and like substituted phenylthioacetamido groups.

Illustrative of the acylamino groups when $R_3$ is a substituted arylalkyl group of the formula

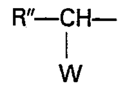

and when W is protected hydroxy are 2-formyloxy-2-phenylacetamido, 2-benzyloxy-2-(4-methoxyphenyl)acetamido, 2-(4-nitrobenzyloxy)-2-(3-chlorophenyl)acetamido, 2-chloroacetoxy-2-(4-methoxyphenyl)acetamido, 2-benzyloxy-2-phenylacetamido 2-trimethylsilyloxy-2-(4-chlorophenyl)acetamido, 2-benzhydryloxy-2-phenylacetamido and like groups. Representative of such groups when W is protected amino are 2-(4-nitrobenzyloxycarbonylamino)-2-phenylacetamido, 2-(2,2,2-trichloroethoxycarbonylamino)-2-phenylacetamido, 2-chloroacetamido-2-(1,4-cyclohexadien-1-yl)acetamido, 2-(4-methoxybenzyloxycarbonylamino)-2-(4-methoxyphenyl)acetamido, 2-benzhydryloxycarbonylamino-2-phenylacetamido, 2-(1-carbomethoxy-2-propenyl)amino-2-phenylacetamido, 2-(4-nitrobenzyloxycarbonylamino)-2-(2-thienyl)acetamido, and like groups. Representative of such groups when W is protected carboxy are 2-(4-methoxybenzyloxycarbonyl)-2-phenylacetamido, 2-benzyloxycarbonyl-2-phenylacetamido, 2-diphenylmethyloxycarbonyl-2-phenylacetamido, 2-(4-nitrobenzyloxycarbonyl)-2-phenylacetamido, 2-(2,2,2-trichloroethoxycarbonyl)2-(2-thienyl)acetamido, 2-(4-methoxybenzyloxycarbonyl)-2-(4-tetrahydropyran-2-ylphenyl)acetamido, and like groups.

Representative of $R_1$ when $R_1$ is an imido group of the formula

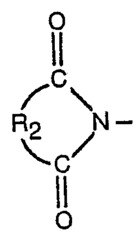

are phthalimido, succinimido, and glutarimido.

Exemplary groups represented by $R_1$ when $R_1$ is an imido group of the formula

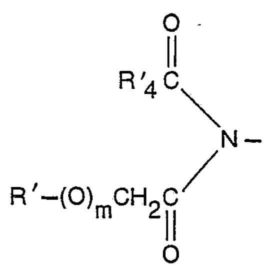

are N-acetyl-N-phenylacetylamino, N-trichloroethoxycarbonyl-N-phenoxyacetylamino, N-propoxycarbonyl-N-(4-chlorophenoxy)acetylamino, N-(2-bromoacetyl)-N-phenoxyacetylamino, and like acyclic imido groups.

Representative of $R_1$ when $R_1$ is an imidazolidinyl group of the formula

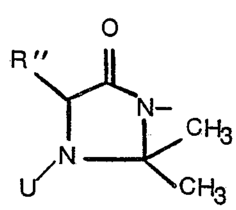

are the 2,2-dimethyl-3-nitroso-5-oxo-4-phenyl-1-imidazolidinyl group, the 2,2-dimethyl-3-nitroso-5-oxo-4-(4-benzyloxyphenyl)-1-imidazolidinyl group, the 2,2-dimethyl-3-acetyl-5-oxo-4-(1,4-cyclohexadien-1-yl)-1-imidazolidinyl group, the 2,2-dimethyl-3-nitroso-5-oxo-4-(2-thienyl)-1-imidazolidinyl group and like substituted imidazolidinyl groups.

The term $R_5$ in the formula 1 represents a conventional carboxy-protecting ester group, for example, those ester groups commonly used to protect carboxylic acid functions in the penicillin and cephalosporin

art. Illustrative of such groups are the alkyl and substituted alkyl ester groups such as t-butyl, 2,2,2-trichloroethyl, 2,2,2-tribromoethyl, 2-iodoethyl, phthalimidomethyl, and the like; the arylalkyl groups such as benzyl, p-nitrobenzyl, p-methoxybenzyl, diphenylmethyl, 4-methoxydiphenylmethyl, methylbenzyl, 3,5-dihydroxybenzyl, and the like. Such esters are employed in this process to prevent the untoward reaction of the carboxylic acid function with the fluorinating reagent.

Examples of amino-protecting groups which can be used in the process to likewise block the free amino function include the conventional amino protecting groups such as those forming urethanes with the amino group eg., t-butyloxycarbonyl, cyclopentyloxycarbonyl, 2,2,2-trichloroethoxycarbonyl, benzyloxycarbonyl, p-nitrobenzyloxycarbonyl, diphenylmethoxycarbonyl, and the like; those forming enamines with β-dicarbonyl compounds eg., methyl acetoacetate and ethyl acetoacetate; and other groups such as the trityl group.

Hydroxy-protecting groups which are conventional and are useful in the process include the halo ester type such as chloroacetyl, trichloroacetyl, dichloroacetyl and bromoacetyl; the ethers such as tetrahydropyranyl, 1-ethoxyethoxy (obtained on protection of the hydroxy group with ethyl vinyl ether), and the like; the silyl groups eg., trialkylsilyl groups such as trimethylsilyl, triethylsilyl, etc.

Other carboxy, amino and hydroxy-protecting groups are well known for example, those described in Chapters 2, 3, and 5, *"Protecting Groups In Organic Chemistry"*, J. F. W. McOmie, Ed., Plenum Press, New York, N.Y. 1973.

Also provided in a process for preparing a 4-fluoroazetidinone of formula 1 as before described which comprises, in any order, reacting in an aprotic inert organic solvent at a temperature between −80°C and −25°C an azetidinone compound of formula 2

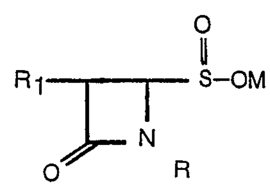

(2)

wherein R is an esterified carboxy substituted butenyl group as above defined and $R_5$ is a carboxy protecting group and $R_1$ is as defined above except that it cannot be amino, and that any hydroxy, carboxy or amino groups are present in protected form, and M is hydrogen, sodium or potassium with perchloryl fluoride; and when desired if R is an esterfied carboxy substituted 3-butenoate isomerizing in the presence of a tertiary amine in an inert hydrocarbon solvent such as methylene chloride, dichloroethane or chloroform; and when the 4-fluoroazetidinone has been prepared removing as desired any hydroxy, carboxy or amino protecting group and/or the acyl group from $R_1$ leaving a 3-aminoazetidinone.

Perchloryl fluoride, $FClO_3$, is a gas under normal conditions of temperature and pressure having a boiling point of about −45°C. The literature sources which describe the preparation and safe handling of this reagent are Fieser and Fieser, *Reagents for Organic Synthesis*, Vol. 1, page 802, and the bulletin, "Perchloryl Fluoride", Pennsalt Chem. Corp.

Since this reagent is a strong oxidizing agent, the term "inert aprotic solvents" refers to solvents which are non-oxidizable by the reagent under the conditions of the process. Aprotic organic solvents which are useful in the process include, for example, dimethylformamide, dimethylacetamide, acetonitrile, tetrahydrofuran, halogenated hydrocarbon solvents such as methylene chloride and chloroform, and like aprotic solvents.

The reaction is carried out by preparing a solution or suspension of the azetidinone-4-sulfinic acid or a salt thereof in an aprotic solvent, which solution is cooled to the temperature of the process and a solution of perchloryl fluoride in an aprotic solvent such as dimethylformamide is added by dropwise addition with stirring to the solution or suspension. Preferably, the process is carried out by employing one molar equivalent of perchloryl fluoride or a slight excess thereof, for example, 1.1 molar equivalents per mole of the azetidinone-4-sulfinic acid.

Alternatively, the cold solution or suspension of the azetidinone sulfinic acid is treated with gaseous perchloryl fluoride by slowly bubbling the gas into the cold solution with stirring.

The process is illustrated by the following reaction scheme wherein $R_1$ is an acylamino group as defined for formula 1.

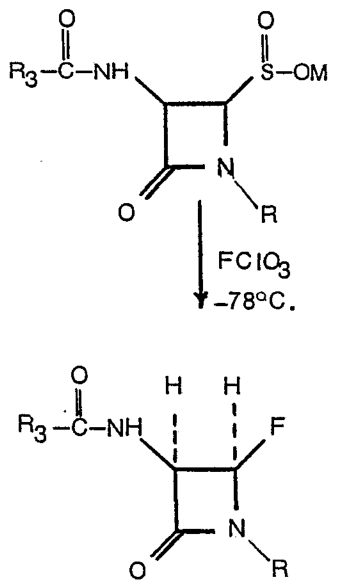


In an example of the process of this invention, p-nitrobenzyl 3-methyl-2-(2-oxo-4-sulfino-3-phenoxyacetamido-1-azetidinyl)-3-butenoate is dissolved in methylene chloride, the solution is cooled to −78°C. in a dry ice-acetone bath and a cold solution of one molar equivalent of perchloryl fluoride in dry dimethylformamide is added by dropwise addition. The reaction proceeds rapidly and is generally complete within 30 minutes, for example at about 20 or 25 minutes.

As shown by the structural formula 1, the *cis*-fluoroazetidinone is the stereochemical isomer obtained by the process of this invention. The acylamino group or the diacylamino group, $R_1$, has the natural or β-configuration, while both protons in the 3- and 4-positions of the β-lactam ring are *cis*.

The compounds of the formula 1 wherein R is a carboxy-substituted or esterified carboxy-substituted butenyl group having the double bond in the α,β-position, as shown in the following formula A, are prepared by the isomerization of the corresponding carboxy-substituted or esterified carboxy-substituted butenyl group represented by the formula B.

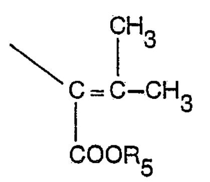

A

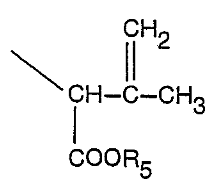

B

The isomerization is carried out by treating a 4-fluoroazetidinone of the formula 1 wherein R is a butenyl group represented by the above formula B with a tertiary amine such as a trialkylamine, for example, triethylamine, in an inert hydrocarbon solvent such as methylene chloride, dichloroethane, or chloroform. The isomerization can be carried out either on the azetidinone-4-sulfinic acid wherein R is a butenyl group having the double bond in the β,γ-position (formula B), or on the corresponding 4-fluoroazetidinone. Accordingly, an azetidinone-4-sulfinic acid wherein the butenyl group, R, is either isomer can be used in the fluorination process of this invention to provide the corresponding 4-fluoroazetidinone. For example, an azetidinone-4-sulfinic acid represented by the formula 1 wherein R is a butenyl group of the formula B is dissolved in methylene chloride and is treated with 2 molar equivalents of triethylamine. The isomerization product, wherein R is a butenyl group of the formula A, above is recovered from the isomerization mixture by washing the reaction mixture with dilute acid to remove the tertiary amine. After drying the reaction mixture, the solvent is evaporated to provide the isomerization product. When an azetidinone-4-sulfinic acid is employed in the isomerization reaction 2 molar equivalents of the tertiary amine or a slight excess thereof are employed. One molar equivalent is tied up by the acidic sulfinic acid group, while the other is employed in the isomerization. When, however, a 4-fluoroazetidinone wherein R is a butenyl group of the formula B is used, the isomerization is carried out with one molar equivalent or a slight excess thereof.

Illustrative compounds of the invention as represented by the formula 1 include the following:

p-nitrobenzyl 3-methyl-2-(2-oxo-4-fluoro-3-cyanoacetamido-1-azetidinyl)-3-butenoate,

p-nitrobenzyl 3-methyl-2-(2-oxo-4-fluoro-3-chloroacetamido-1-azetidinyl)-3-butenoate,

diphenylmethyl 3-methyl-2-(2-oxo-4-fluoro-3-acetamido-1-azetidinyl)-3-butenoate,
p-methoxybenzyl 3-methyl-2-(2-oxo-4-fluoro-3-p-methoxybenzyloxycarbonylamino-1-azetidinyl)-3-butenoate,
p-nitrobenzyl 3-methyl-2-(2-oxo-4-fluoro-3-t-butyloxycarbonylamino-1-azetidinyl)-2-butenoate,
p-nitrobenzyl 3-methyl-2-(2-oxo-4-fluoro-3-phthalimido-1-azetidinyl)-3-butenoate,
p-nitrobenzyl 3-methyl-2-(2-oxo-4-fluoro-3-succinimido-1-azetidinyl)-2-butenoate,
diphenylmethyl 3-methyl-2-(2-oxo-4-fluoro-3-benzamido-1-azetidinyl)-3-butenoate,
diphenylmethyl 3-methyl-2-(2-oxo-4-fluoro-3-p-toluamido-1-azetidinyl)-3-butenoate,
3-methyl-2-(2-oxo-4-fluoro-3-p-toluamido-1-azetidinyl)-3-butenoic acid,
diphenylmethyl 3-methyl-2-(2-oxo-4-fluoro-3-phenylacetamido-1-azetidinyl)-3-butenoate,
diphenylmethyl 3-methyl-2-(2-oxo-4-fluoro-3-phenylacetamido-1-azetidinyl)-2-butenoate,
3-methyl-2-(2-oxo-4-fluoro-3-phenylacetamido-1-azetidinyl)-3-butenoic acid,
p-nitrobenzyl 3-methyl-2-(2-oxo-4-fluoro-3-phenoxyacetamido-1-azetidinyl)-3-butenoate,
p-nitrobenzyl 3-methyl-2-(2-oxo-4-fluoro-3-phenoxyacetamido-1-azetidinyl)-2-butenoate,
3-methyl-2-(2-oxo-4-fluoro-3-phenoxyacetamido-1-azetidinyl)-3-butenoic acid,
2,2,2-trichloroethyl 3-methyl-2-[2-oxo-4-fluoro-3-(2-thienylacetamido)-1-azetidinyl]-3-butenoate,
p-nitrobenzyl 3-methyl-2-(2-oxo-4-fluoro-3-phenylmercaptoacetamido-1-azetidinyl)-3-butenoate,
3-methyl-2-(2-oxo-3-fluoro-3-phenylglycylamino-1-azetidinyl)-2-butenoic acid,
3-methyl-2-[2-oxo-4-fluoro-3-(2-hydroxy-2-phenylacetamido)-1-azetidinyl]-2-butenoic acid, and
3-methyl-2-[2-oxo-4-fluoro-3-(2-carboxy-2-phenylacetamido)-1-azetidinyl]-2-butenoic acid.

The compounds of the invention where in formula 1 $R_1$ is an amino group, are prepared by the N-deacylation of a 3-acylamino-4-fluoroazetidinone. The N-deacylation is carried out by the well known two-step N-deacylation process commonly employed in the cephalosporin and penicillin arts for the preparation of the 7-aminocephalosporin nucleus and the 6-aminopenicillin nucleus. According to the method, a 3-acylamino-4-fluoroazetidinone is reacted with an imino halide forming reagent such as phosphorus pentachloride in an inert solvent, for example, a chlorinated hydrocarbon solvent such as methylene chloride, dichloroethane or trichloroethane at a temperature between −15°C. and 25°C. to form the corresponding imino chloride. The imino chloride intermediate is converted to the imino ether by treatment of the imino chloride reaction mixture with an alcohol such as methanol or isobutanol. The formation of the imino ether is carried out by first lowering the temperature of the imino chloride reaction mixture to a temperature of about −25°C. followed by addition of at least one molar equivalent of the alcohol. When imino ether formation is complete, the reaction mixture is allowed to warm to room temperature and the imino ether is hydrolyzed by the addition of water to the reaction mixture. The 3-amino-4-fluoroazetidinone of the formula 1 is best recovered from the reaction mixture as the hydrochloride salt. The preparation of the 3-amino compounds of the formula 1 is illustrated by the following reaction scheme.

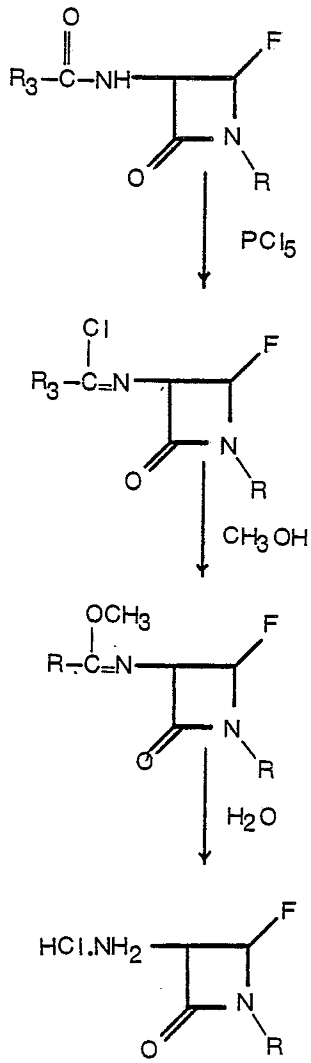


wherein R is

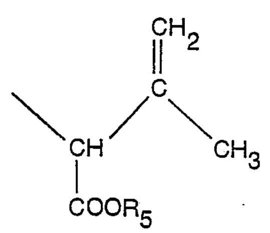

or

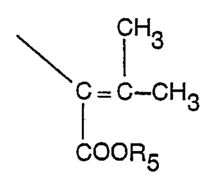

and $R_5$ is a protected carboxy group.

The 3-amino-4-fluoroazetidinones are useful intermediates for the preparation of the 3-acylamino-azetidinones of the formula 1. For example, the 3-amino compounds can be acylated with the desired acyl group to provide a compound of the formula 1 wherein $R_1$ is an acylamido or diacylamido group.

The 3-amino-4-fluoroazetidinones are also obtained by the alternate route comprising the cleavage of a compound of the formula 1 wherein $R_3$ is benzyloxy, p-nitrobenzyloxy, t-butyloxy, 2,2,2-trichloroethoxy, or p-methoxybenzyloxy. These urethane-forming groups are readily removed by known procedures, for example, with a mild acid such as hydrochloric acid or hydrobromic acid. The benzyloxy and substituted benzyloxy groups can be removed also by catalytic hydrogenolysis over palladium on carbon.

The 3-aminoazetidinones of the formula 1 can be obtained as the free amines or as salts formed with mineral acids, for example, hydrochloric acid, hydrobromic acid, sulfuric acid, and phosphoric acid. The salts are formed in a conventional manner, for example, by treating a solution of the free aminoazetidinone in a solvent such as acetone or an alcohol such as ethyl alcohol with an equivalent or a slight excess of the acid. Examples of 3-amino-4-fluoroazetidinones represented by the formula 1 include the following:

p-nitrobenzyl 3-methyl-2-(2-oxo-4-fluoro-3-amino-1-azetidinyl)-3-butenoate,

p-nitrobenzyl 3-methyl-2-(2-oxo-4-fluoro-3-amino-1-azetidinyl)-2-butenoate,

diphenylmethyl 3-methyl-2-(2-oxo-4-fluoro-3-amino-1-azetidinyl)-3-butenoate,
diphenylmethyl 3-methyl-2-(2-oxo-4-fluoro-3-amino-1-azetidinyl)-2-butenoate,
p-methoxybenzyl 3-methyl-2-(2-oxo-4-fluoro-3-amino-1-azetidinyl)-3-butenoate,
t-butyl 3-methyl-2-(2-oxo-4-fluoro-3-amino-1-azetidinyl)-3-butenoate,
2,2,2-trichloroethyl 3-methyl-2-(2-oxo-4-fluoro-3-amino-1-azetidinyl)-2-butenoate,
3-methyl-2-(2-oxo-4-fluoro-3-amino-1-azetidinyl)-3-butenoic acid,
3-methyl-2-(2-oxo-4-fluoro-3-amino-1-azetidinyl)-2-butenoic acid, and the acid addition salts thereof formed with mineral acids.

The compounds of the invention represented by the following formula

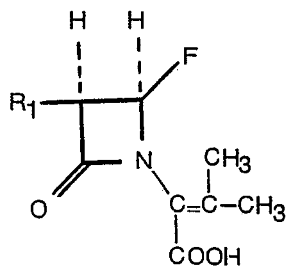

3

and the pharmaceutically acceptable salts thereof wherein $R_1$ is other than amino, benzyloxycarbonylamino, p-nitrobenzyloxycarbonylamino, t-butyloxycarbonylamino, 2,2,2-trichloroethoxycarbonylamino, or p-methoxybenzyloxycarbonylamino are antibacterial agents which inhibit the growth of microorganisms pathogenic to man and animals. These compounds can be used as topical sterilants and can be formulated at suitable concentrations in liquid form for example, as an aqueous solution or emulsion containing between about 1% and 25% of the active antibacterial agent. The compounds are preferably used in salt form such as the sodium or potassium salt. Other pharmaceutically acceptable salts which can be used include the calcium salt, the aluminum salt, and salts formed with organic amines such as ethanolamine, propanolamine, dibenzylamine, dicyclohexylamine, abietylamine, and like amines.

Antibacterial formulations comprising as an active ingredient 1 to 25% by weight of a 4-fluoroazetidinone of formula 3 as described above and one or more pharmaceutically acceptable carriers or adjuvants are contemplated.

Preferred compounds represented by the above formula 3 are those wherein $R_1$ is an acylamino group, $R_3$—C(O)NH—. Preferred compounds of this group include those wherein $R_3$ is phenyl or substituted phenyl as defined hereinabove, benzyl, substituted benzyl, phenoxymethyl, and 2-thienyl.

The compounds of the formula 1 wherein $R_5$ is a carboxy-protecting ester group are useful intermediates in the preparation of the above antibacterial compounds. Likewise, the compounds of the formula 1 wherein R is a butenyl group of the above formula B wherein the double bond is in the β,γ-position are intermediates which can be isomerized by the above-described method to the antibacterials of the above formula 3 or to intermediates thereof.

The starting materials employed in the process of this invention, namely, azetidin-2-one-4-sulfinic acids of the above formula 2, are known compounds and are described by Kukolja in U.S. Patent No. 4,159,266. The azetidinone-4-sulfinic acids are prepared from the corresponding sulfinyl chlorides represented by the following formula

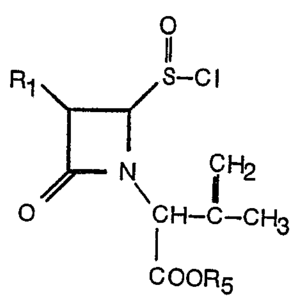

wherein $R_1$ and $R_5$ have the same meanings as defined for formulas 1 and 2 hereinabove. The sulfinyl chloride is dissolved in a suitable water immiscible organic solvent such as ethyl acetate and the solution is slurried with an aqueous solution of sodium bicarbonate. The aqueous layer containing the sulfinic acid sodium salt is separated, washed with ethyl acetate and relayered with fresh ethyl acetate and then acidified. The organic layer containing the sulfinic acid is separated, washed and evaporated to dryness to provide the sulfinic acid as an amorphous solid.

The sodium salts of some of the sulfinic acids used in the invention are sufficiently soluble in ethyl acetate such that only minor amounts will partition into water as described above. In these instances the

hydrolysis can be carried out using toluene or alternatively, the azetidinone sulfinyl chloride can be hydrolyzed in an aromatic hydrocarbon such as toluene with 1 N hydrochloric acid.

Examples of sulfinic acids useful as starting materials in the process of this invention are the following wherein the formal names are used:

p-nitrobenzyl 3-methyl-2-(2-oxo-4-sulfino-3-phenylacetamido-1-azetidinyl)-3-butenoate,
p-methoxybenzyl 3-methyl-2-(2-oxo-4-sulfino-3-phenoxyacetamido-1-azetidinyl)-3-butenoate,
diphenylmethyl 3-methyl-2-[2-oxo-4-sulfino-3-(4-methylbenzamido)-1-azetidinyl]-3-butenoate,
2,2,2-trichloroethyl 3-methyl-2-(2-oxo-4-sulfino-3-chloroacetamido-1-azetidinyl)-3-butenoate,
p-nitrobenzyl 3-methyl-2-[2-oxo-4-sulfino-3-(4-nitrobenzyloxycarbonylamino)-1-azetidinyl]-3-butenoate,
2,2,2-trichloroethyl 3-methyl-2-[2-oxo-4-sulfino-3-(2-thienylacetamido)-1-azetidinyl]-3-butenoate,
diphenylmethyl 3-methyl-2-[2-oxo-4-sulfino-3-(2-p-methoxybenzyloxycarbonyl-2-phenylacetamido)-1-azetidinyl]-3-butenoate,
2-iodoethyl 3-methyl-2-(2-oxo-4-sulfino-3-benzamido-1-azetidinyl)-3-butenoate, and
p-methoxybenzyl 3-methyl-2-(2-oxo-4-sulfino-3-phthalimido-1-azetidinyl)-3-butenoate.

The azetidinone sulfinyl chlorides which on hydrolysis form the corresponding sulfinic acids as described above are prepared as described by Kukolja, U.S. Patent No. 4,081,440.

Examples of 4-chlorosulfinylazetidinones are represented by the following formula

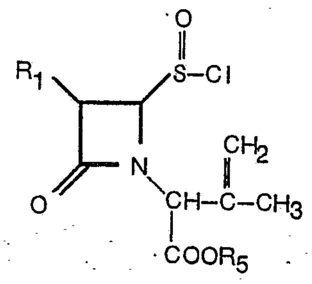

wherein $R_1$ and $R_5$ are as follows:

| $R_1$ | $R_5$ |
|---|---|
| phenoxyacetamido | p-nitrobenzyl |
| phenoxyacetamido | diphenylmethyl |
| phenoxyacetamido | p-methoxybenzyl |
| phenoxyacetamido | 2,2,2-trichloroethyl |
| phenylacetamido | diphenylmethyl |
| cyanoacetamido | benzyl |
| chloroacetamido | t-butyl |
| phthalimido | p-nitrobenzyl |
| succinimido | benzyl |
| 2-thienylacetamido | 2,2,2-trichloroethyl |
| 3-thienylacetamido | 2-iodoethyl |
| 2-furylacetamido | t-butyl |
| benzamido | diphenylmethyl |
| p-methylbenzamido | diphenylmethyl |
| p-chlorobenzamido | diphenylmethyl |
| acetamido | p-nitrobenzyl |

| $R_1$ | $R_5$ |
|---|---|
| 2-(p-methoxybenzyloxy-carbonyl)-2-phenyl-acetamido | diphenylmethyl |
| p-chlorophenylthio-acetamido | 2,2,2-trichloroethyl |
| p-chlorophenoxyacetamido | p-nitrobenzyl |
| 2-(t-butyloxycarbonyl-amino)-2-phenylacetamido | diphenylmethyl |
| 2-(tetrahydropyran-2-yl)-2-phenylacetamido | p-nitrobenzyl |
| phenylthioacetamido | p-methoxybenzyl |

As mentioned above the 4-chlorosulfinylazetidinones are known compounds and can be obtained by the method described in U.S. Patent No. 4,081,440, or as described by Chow in U.S. Patent No. 4,075,203 using an epoxide or calcium oxide as a non-alkaline acid scavenger. Likewise, Chow describes the use of poly(4-vinylpyridine)-divinylbenzene copolymer crosslinked to about 2% as a preferred acid scavenger in the process for preparing the chlorosulfinylazetidinones with N-chloro halogenating agents, U.S. Patent No. 4,190,724.

The following examples further illustrate the compounds of the invention and the process for the preparation thereof. In the examples, the following abbreviations are used: pNB = p-nitrobenzyl; pMB = p-methoxybenzyl; DPM = diphenylmethyl.

## Example 1

3-Methyl-2-(2-oxo-4-fluoro-3-phenoxyacetamido-1-azetidinyl)-3-butenoic acid

A solution of 2.9 g. (5 mmoles) of p-nitrobenzyl 3-methyl-2-(2-oxo-4-sulfino-3-phenoxyacetamido-1-azetidinyl)-3-butenoate in 100 ml. of methylene chloride was cooled to −78°C. in a dry ice-acetone bath and a solution of 5 mmoles of perchloroyl fluoride in 105 ml. of dry DMF was added by dropwise addition over five minutes. After addition was completed the reaction mixture was stirred in the cold for 20 minutes and was then poured into a mixture of 500 ml. of an aqueous saturated sodium chloride solution and 250 ml. of ethyl acetate. The mixture was shaken, the organic layer separated and washed with aqueous brine and aqueous sodium bicarbonate solution and was dried. The dried solution of the product was evaporated to dryness and the residue of product was mixed with a small volume of ethyl acetate. The product, p-nitrobenzyl 3-methyl-2-(2-oxo-4-fluoro-3-phenoxyacetamido-1-azetidinyl)-3-butenoate, crystallized and was filtered and dried.

IR ($CHCl_3$): 1795 $cm^{-1}$; β-lactam carbonyl.

NMR ($CDCl_3$) δ: 1.87 (s, 3H, $CH_3$), 4.56 (s, 2H, $CH_2O$O), 5.01 and 5.18 (m, 2H, $CH_2$=), 5.05 (s, 1H, CHCoopNB), 5.31 (s, 2H, $CH_2$ of pNB), 5.55 (m, 1H, azetidinone H), 5.90 and 6.66 (dd, 1H, azetidinone H), 6.85—8.34 (m, 10H, aromatic H and NH).

To a suspension of 250 mg. of 5% palladium on carbon catalyst in 25 ml. of tetrahydrofuran prehydrogenated on a Parr Hydrogenation apparatus were added a solution of 250 mg. of the 4-fluoroazetidinone prepared as described in the minimum amount of tetrahydrofuran. The suspension was hydrogenated for about 4 hours and was filtered. The filtrate was evaporated under reduced pressure and the residue of product obtained dissolved in ethyl acetate. Water was added to the solution and the pH was adjusted to 7.0. The aqueous phase was separated, layered with fresh ethyl acetate and the pH adjusted to 2.0. The ethyl acetate layer containing the title compound was separated, dried over magnesium sulfate, and evaporated to dryness. The title compound was obtained as a white foam.

## Example 2

3-Methyl-2-(2-oxo-4-fluoro-3-phenoxyacetamido-1-azetidinyl)-2-butenoic acid

The 4-fluoroazetidinone ester prepared as described in Example 1 was dissolved in methylene chloride and triethylamine was added with stirring at room temperature. After 30 minutes the isomerization mixture was evaporated to dryness removing the solvent and amine and leaving the isomerization product, p-nitrobenzyl 3-methyl-2-(2-oxo-4-fluoro-3-phenoxyacetamido-1-azetidinyl)-2-butenoate as residue.

The residue of product was dissolved in THF and the solution added to a suspension of 5% palladium on carbon catalyst in THF. The mixture was hydrogenated as described in Example 1 and the title compound was obtained.

Example 3

By following the procedures and conditions described in Example 1, diphenylmethyl 3-methyl-2-(2-oxo-4-fluoro-3-phenylacetamido-1-azetidinyl)-3-butenoate is obtained by reacting the corresponding 4-sufinoazetidinone with perchloroyl fluoride in methylene chloride. The 4-fluoro product is isomerized in methylene chloride with triethylamine and the isomerization product is deesterified with trifluoroacetic acid containing anisole at 5°C. to 3-methyl-2-(2-oxo-4-fluoro-3-phenylacetamido-1-azetidinyl)-2-butenoic acid.

Example 4

By following the procedures and conditions described in Example 1, diphenylmethyl 3-methyl-2-(2-oxo-4-fluoro-3-p-toluamido-1-azetidinyl)-3-butenoate is obtained by reacting diphenylmethyl 3-methyl-2-(2-oxo-4-sulfino-3-p-toluamido-1-azetidinyl)-3-butenoate with perchloroyl fluoride.

Example 5

2,2,2-Trichloroethyl 3-methyl-2-[2-oxo-4-fluoro-3-(2-thienylacetamido)-1-azetidinyl]-3-butenoate is obtained by reacting 2,2,2-trichloroethyl 3-methyl-2-[2-oxo-4-sulfino-3-(2-thienylacetamido)-1-azetidinyl]-3-butenoate with perchloroyl fluoride under the conditions described in Example 1. The product is isomerized and deesterified to 3-methyl-2-[2-oxo-4-fluoro-3-(2-thienylacetamido)-1-azetidinyl]-2-butenoic acid.

Example 6

p-Methoxybenzyl 3-methyl-2-[2-oxo-4-fluoro-3-(benzyloxycarbonylamino)-1-azetidinyl]-3-butenoate is obtained by reacting the correspondingly substituted azetidinone-4-sulfinic acid with perchloroyl fluoride in 1,1,2-trichloroethane. The product is hydrogenated under 3.45 + $10^5$ Pa (50 psi) hydrogen pressure in the presence of 5% palladium on carbon catalyst to produce p-methoxybenzyl 3-methyl-2-(2-oxo-4-fluoro-3-amino-1-azetidinyl)-3-butenoate.

Example 7

p-Nitrobenzyl 3-methyl-2-(2-oxo-4-fluoro-3-phthalimido-1-azetidinyl)-3-butenoate is obtained by reacting the correspondingly substituted azetidinone-4-sulfinic acid in chloroform with perchloryl fluoride at −78°C.

Example 8

t-Butyl 3-methyl-2-(2-oxo-4-fluoro-3-acetamido-1-azetidinyl)-3-butenoate is obtained by reacting the correspondingly substituted azetidinone-4-sulfinic acid with perchloryl fluoride in methylene chloride at −78°C.

Example 9

p-Nitrobenzyl 3-methyl-2-[2-oxo-4-fluoro-3-2-t-butyloxycarbonylamino-2-phenylacetamido)-1-azetidinyl]-3-butenoate is obtained by reacting the correspondingly substituted azetidinone-4-sulfinic acid with perchloryl fluoride in methylene chloride at −78°C. The product is isomerized with triethylamine in methylene chloride to the 2-butenoate and on treatment with trifluoroacetic acid the t-butyloxycarbonyl group is removed. The p-nitrobenzyl ester is next removed by treating the ester with zinc and acetic acid in DMF. There is obtained 3-methyl-2-[2-oxo-4-fluoro-3-(2-amino-2-phenylacetamido)-1-azetidinyl]-2-butenoic acid represented by the formula.

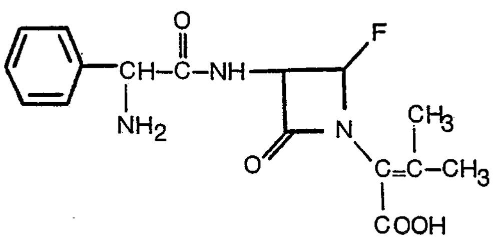

**Claims**

1. A 4-fluoroazetidinone of formula 1

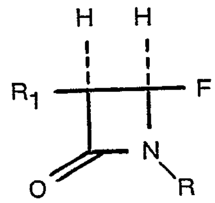

(1)

wherein $R_1$ is amino or

1) an imido group of the formula

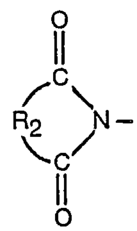

wherein $R_2$ is $C_2$—$C_4$ alkylene or 1,2-phenylene;

2) an amido group of the formula

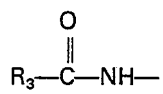

wherein $R_3$ is

a) hydrogen, $C_1$—$C_4$ alkyl, halomethyl, cyanomethyl, benzyloxy, p-nitrobenzyloxy, t-butyloxy, 2,2,2-trichloroethoxy, or p-methoxybenzyloxy;

b) the group R′, wherein R′ in phenyl or phenyl substituted by 1 or 2 halogens, hydroxy, protected hydroxy, nitro, cyano, $C_1$—$C_4$ alkyl, or $C_1$—$C_4$ alkoxy;

c) a group of the formula

$$R''—(Q)_m—CH_2—$$

wherein R″ is R′ as defined above, 1,4-cyclohexadienyl, thienyl or furyl; m is 0 or 1; and Q is O or S; with the limitation that when m is 1, R″ is R′;

d) a group of the formula

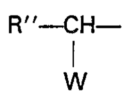

wherein R″ is as defined above, and W is hydroxy, protected hydroxy, carboxy, protected carboxy, amino, or protected amino;

3) an imidazolidinyl group of the formula

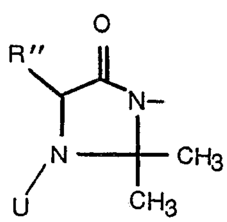

wherein R″ is as defined above and U is nitroso or acetyl; or $R_1$ is

4) an imido group of the formula

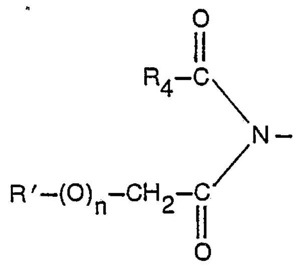

wherein R′ is as defined above, n is 0 or 1, and $R_4$ is $C_1$—$C_4$ alkyl, $C_1$—$C_4$ haloalkyl, $C_1$—$C_4$ alkoxy, or 2,2,2-trichloroethoxy; R is a carboxy-substituted or esterified carboxy-substituted butenyl group represented by the formulas

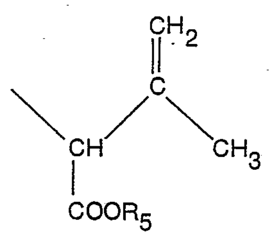 , 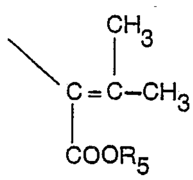

wherein $R_5$ is hydrogen or a carboxy protecting group; or a pharmaceutically-acceptable salt thereof.

2. The compound of claim 1 wherein $R_1$ is other than amino, benzyloxycarbonylamino, p-nitrobenzyloxycarbonylamino, t-butyloxycarbonylamino, 2,2,2-trichloroethoxycarbonylamino, or p-methoxybenzyloxycarbonylamino, and R is the carboxy-substituted butenyl group having the double bond α,β.

3. The compound of claim 1 wherein R is a group of the formula

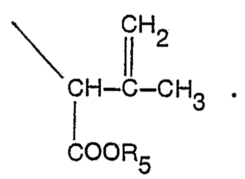

4. The compound of claim 1 wherein R is a group of the formula

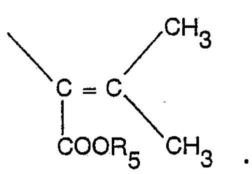

5. The compound of any one of claims 1, 3 and 4 wherein $R_1$ is a group of the formula

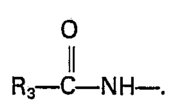

6. A 4-fluoroazetidinone 3-butenoic acid derivative of formula 1 as claimed in claim 1 which is selected from the group:

p-nitrobenzyl 3-methyl-2-(2-oxo-4-fluoro-3-phenoxyacetamido-1-azetidinyl)-3-butenoate,
3-methyl-2-(2-oxo-4-fluoro-3-phenoxyacetamido-1-azetidinyl)-3-butenoic acid,
diphenylmethyl 3-methyl-2-(2-oxo-4-fluoro-3-phenylacetamido-1-azetidinyl)-3-butenoate,
3-methyl-2-(2-oxo-4-fluoro-3-phenyl-acetamido-1-azetidinyl)-3-butenoic acid,
diphenylmethyl 3-methyl-2-(2-oxo-4-fluoro-3-p-toluamido-1-azetidinyl)-3-butenoate,
2,2,2-trichloroethyl 3-methyl-2-[2-oxo-4-fluoro-3-(2-thienylacetamido)-1-azetidinyl]-3-butenoate,
p-methoxybenzyl 3-methyl-2-[2-oxo-4-fluoro-3-(benzyloxycarbonylamino)-1-azetidinyl]-3-butenoate,
p-methoxybenzyl 3-methyl-2-(2-oxo-4-fluoro-3-amino-1-azetidinyl)-3-butenoate,

p-nitrobenzyl 3-methyl-2-(2-oxo-4-fluoro-3-phthalimido-1-azetidinyl)-3-butenoate,
t-butyl 3-methyl-2-(2-oxo-4-fluoro-3-acetamido-1-azetidinyl)-3-butenoate, and
p-nitrobenzyl 3-methyl-2-[2-oxo-4-fluoro-3-(2-t-butyloxycarbonylamino-2-phenylacetamido)-1-azetidinyl]-3-butenoate.

7. A 4-fluoroazetidinone 2-butenoic acid derivative of formula 1 as claimed in claim 1 which is selected from the group
p-nitrobenzyl 3-methyl-2-(2-oxo-4-fluoro-3-phenoxyacetamido-1-azetidinyl)-2-butenoate,
3-methyl-2-(2-oxo-4-fluoro-3-phenoxyacetamido-1-azetidinyl)-2-butenoic acid,
2,2,2-trichloroethyl-3-methyl-2-[2-oxo-4-fluoro-3-(2-thienylacetamido)-1-azetidinyl]-2-butenoate,
3-methyl-2-[2-oxo-4-fluoro-3-(2-thienylacetamido)-1-azetidinyl]-2-butenoic acid and
3-methyl-2-[2-oxo-4-fluoro-3-(2-amino-2-phenylacetamido)-1-azetidinyl]-2-butenoic acid.

8. A pharmaceutically-acceptable salt of a 4-fluoroazetidinone of formula 1 as claimed in claim 2.

9. An antibacterial formulation comprising as an active ingredient 1 to 25% by weight of a 4-fluoroazetidinone of formula 1 wherein $R_1$ is as described above other than amino, benzyloxycarbonylamino, p-nitrobenzyloxycarbonylamino, t-butyloxycarbonylamino, 2,2,2-trichloroethoxycarbonylamino, or p-methoxybenzyloxycarbonylamino; and R is a carboxy-substituted butenyl represented by the formula

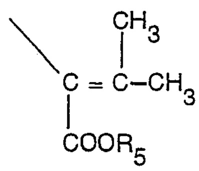

and $R_5$ is hydrogen and one or more pharmaceutically acceptable carriers or adjuvents.

10. A process for preparing a 4-fluoroazetidinone of formula 1 as described in any one of claims 1 to 8 which comprises, in any order, reacting in an aprotic inert organic solvent at a temperature between −80°C and −25°C an azetidinone compound of formula 2

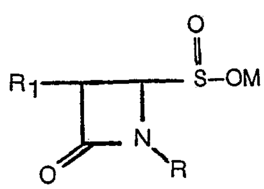

(2)

wherein R is an esterfied carboxy substituted butenyl group as above defined and $R_5$ is a carboxy protecting group and $R_1$ is as defined in claim 1 except that it cannot be amino, and that any hydroxy, carboxy or amino groups are present in protected form, and M is hydrogen, sodium or potassium with perchloryl fluoride; and when desired if R is an esterified carboxy substituted 3-butenoate isomerizing in the presence of a tertiary amine in an inert hydrocarbon solvent such as methylene chloride, dichloroethane or chloroform; and when the 4-fluoroazetidinone has been prepared removing as desired any hydroxy, carboxy or amino protecting group and/or the acyl group from $R_1$ leaving a 3-aminoazetidinone.

11. A process of preparing p-nitrobenzyl 3-methyl-2-(2-oxo-4-fluoro-3-phenoxyacetamido-1-azetidinyl)-3-butenoate as claimed in claim 6 which comprises reacting a solution of p-nitrobenzyl 3-methyl-2-(2-oxo-4-sulfino-3-phenoxyacetamido-1-azetidinyl)-3-butenoate in methylene chloride at −78°C with perchloryl fluoride in dimethylformamide for about 25 minutes.

12. A process of preparing p-nitrobenzyl 3-methyl-2-(2-oxo-4-fluoro-3-phenoxyacetamido-1-azetidinyl)-2-butenoate as claimed in claim 7 which comprises isomerizing p-nitrobenzyl 3-methyl-2-(2-oxo-4-fluoro-3-phenoxyacetamido-1-azetidinyl)-3-butenoate, prepared by the proccess of claim 11, in methylene chloride in the presence of triethylamine with stirring at room temperature for thirty minutes.

**Patentansprüche**

1. 4-Fluorazetidinon der Formel 1

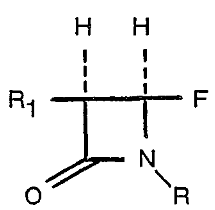

(1)

worin $R_1$ Amino ist oder folgende Bedeutungen hat

1) eine Imidogruppe der Formel

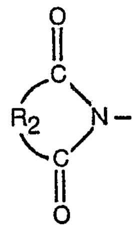

worin $R_2$ für $C_2$—$C_4$-Alkylen oder 1,2-Phenylen steht,

2) eine Amidogruppe der Formel

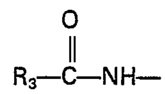

worin $R_3$ folgende Bedeutungen hat

a) Wasserstoff, $C_1$—$C_4$-Alkyl, Halogenmethyl, Cyanomethyl, Benzyloxy, p-Nitrobenzyloxy, t-Butyloxy, 2,2,2-Trichlorethoxy oder p-Methoxybenzyloxy,

b) die Gruppe R′, worin R′ Phenyl oder einfach oder zweifach durch Halogen, Hydroxy, geschütztes Hydroxy, Nitro, Cyano, $C_1$—$C_4$-Alkyl oder $C_1$—$C_4$-Alkoxy substituiertes Phenyl ist,

c) eine Gruppe der Formel

$$R''—(Q)_m—CH_2—,$$

worin R″ für R′ gemäß obiger Definition, 1,4-cyclohexadienyl, Thienyl oder Furyl steht, m für 0 oder 1 steht und Q für O oder S steht, mit der Maßgabe, daß R″ für R′ steht, falls m für 1 steht,

d) eine Gruppe der Formel

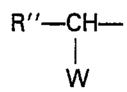

worin R″ wie oben definiert ist und W Hydroxy, geschütztes Hydroxy, Carboxy, geschütztes Carboxy, Amino oder geschütztes Amino bedeutet,

3) eine Imidazolidinylgruppe der Formel

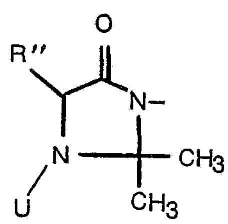

worin R″ wie oben definiert ist und U für Nitroso oder Acetyl steht, oder

4) eine Imidogruppe der Formel

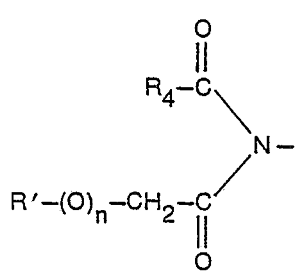

worin R′ wie oben definiert ist, n für 0 oder 1 steht und $R_4$ für $C_1$—$C_4$-Alkyl, $C_1$—$C_4$-Halogenalkyl, $C_1$—$C_4$-Alkoxy oder 2,2,2-Trichlorethoxy steht, R eine carboxysubstituierte oder veresterte carboxysubstituierte Butenylgruppe der Formeln

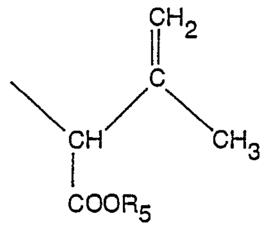

oder

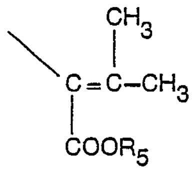

bedeutet, worin $R_5$ Wasserstoff oder eine Carboxyschutzgruppe ist, oder ein pharmazeutisch annehmbares Salz hiervon.

2. Verbindung nach Anspruch 1, dadurch gekennzeichnet, daß $R_1$ eine andere Bedeutung hat als Amino, Benzyloxycarbonylamino, p-Nitrobenzyloxycarbonylamino, t-Butyloxycarbonylamino, 2,2,2-Trichlorethoxycarbonylamino oder p-Methoxybenzyloxycarbonylamino und R für die carboxysubstituierte Butenylgruppe mit der Doppelbindung in Stellung α,β steht.

3. Verbindung nach Anspruch 1, dadurch gekennzeichnet, daß R eine Gruppe der Formel

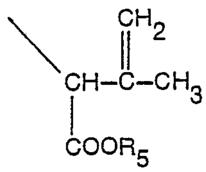

ist.

4. Verbindung nach Anspruch 1, dadurch gekennzeichnet, daß R eine Gruppe der Formel

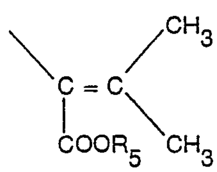

ist.

5. Verbindung nach einem der Ansprüche 1, 3 oder 4, dadurch gekennzeichnet, daß $R_1$ eine Gruppe der Formel

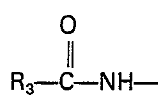

ist.

6. 4-Fluorazetidinon-3-butencarbonsäure-Derivat der Formel 1 nach Anspruch 1, dadurch gekennzeichnet, daß es sich hierbei um eine der folgenden Verbindungen handelt:

p-Nitrobenzyl-3-methyl-2-(2-oxo-4-fluor-3-phenoxyacetamido-1-azetidinyl)-3-butenoat,
3-Methyl-2-(2-oxo-4-fluor-3-phenoxyacetamido-1-azetidinyl)-3-butencarbonsäure,
Diphenylmethyl-3-methyl-2-(2-oxo-4-fluor-3-phenylacetamido-1-azetidinyl)-3-butenoat,
3-Methyl-2-(2-oxo-4-fluor-3-phenyl-acetamido-1-azetidinyl)-3-butencarbonsäure,
Diphenylmethyl-3-methyl-2-(2-oxo-4-fluor-3-p-toluamido-1-azetidinyl)-3-butenoat,
2,2,2-Trichlorethyl-3-methyl-2-[2-oxo-4-fluor-3-(2-thienylacetamido)-1-azetidinyl]-3-butenoat,
p-Methoxybenzyl-3-methyl-2-[2-oxo-4-fluor-3-(benzyloxycarbonylamino)-1-azetidinyl]-3-butenoat,
p-Methoxybenzyl-3-methyl-2-(2-oxo-4-fluor-3-amino-1-azetidinyl)-3-butenoat,
p-Nitrobenzyl-3-methyl-2-(2-oxo-4-fluor-3-phthalimido-1-azetidinyl)-3-butenoat,
t-Butyl-3-methyl-2-(2-oxo-4-fluor-3-acetamido-1-azetidinyl)-3-butenoat und
p-Nitrobenzyl-3-methyl-2-[2-oxo-4-fluor-3-(2-t-butyloxycarbonylamino-2-phenylacetamido)-1-azetidinyl]-3-butenoat.

7. 4-Fluorazetidinon-2-butencarbonsäure-Derivat der Formel 1 nach Anspruch 1, dadurch gekennzeichnet, daß es sich dabei um eine der folgenden Verbindungen handelt:

p-Nitrobenzyl-3-methyl-2-(2-oxo-4-fluor-3-phenoxyacetamido-1-azetidinyl)-2-butenoat,
3-Methyl-2-(2-oxo-4-fluor-3-phenoxyacetamido-1-azetidinyl)-2-butencarbonsäure,
2,2,2-Trichlorethyl-3-methyl-2-[2-oxo-4-fluor-3-(2-thienylacetamido)-1-azetidinyl]-2-butenoat,
3-Methyl-2-[2-oxo-4-fluor-3-(2-thienylacetamido)-1-azetidinyl]-2-butencarbonsäure und

3-Methyl-2-[2-oxo-4-fluor-3-(2-amino-2-phenylacetamido)-1-azetidinyl]-2-butencarbonsäure.

8. Pharmazeutisch annehmbares Salz eines 4-Fluorazetidinons der Formel 1 nach Anspruch 2.

9. Antibakterielle Formulierung, gekennzeichnet durch einen Gehalt an 1 bis 25 Gew.-% eines 4-Fluorazetidinons der Formel 1, worin $R_1$ eine andere Bedeutung als Amino, Benzyloxycarbonylamino, p-Nitrobenzyloxycarbonylamino, t-Butyloxycarbonylamino, 2,2,2-Trichlorethoxycarbonylamino oder p-Methoxybenzyloxycarbonylamino hat und R die carboxysubstituierte Butenylgruppe der Formel

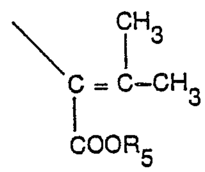

ist, worin $R_5$ Wasserstoff bedeutet, als Wirkstoff in Verbindung mit einem oder mehreren pharmazeutisch annehmbaren Trägern oder Hilfsstoffen hierfür.

10. Verfahren zur Herstellung eines 4-Fluorazetidinons der Formel 1 nach einem der Ansprüche 1 bis 8, dadurch gekennzeichnet, daß man in beliebiger Reihenfolge in einem aprotischen inerten organischen Lösungsmittel bei einer Temperatur zwischen −80°C und −25°C eine Azetidinonverbindung der Formel 2

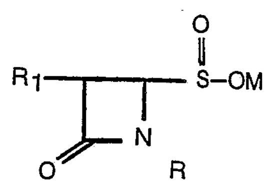

(2)

worin R eine veresterte carboxysubstituierte Butenylgruppe gemäß obiger Definition ist, wobei $R_5$ eine Carboxyschutzgruppe bedeutet, $R_1$ die im Anspruch 1 angegebene Definition mit der Ausnahme hat, daß dieser Rest nicht für Amino stehen kann und daß irgendwelche eventuelle vorhandenen Hydroxy-, Carboxy- oder Aminogruppen in geschützter Form vorliegen, und M Wasserstoff, Natrium oder Kalium bedeutet, mit Perchlorylfluorid umsetzt, und, falls R ein verestertes carboxysubstituiertes 3-Butenoat ist, gegebenenfalls eine entsprechende Verbindung in Gegenwart eines tertiären Amins in einem inerten Kohlenwasserstoff als Lösungsmittel, wie Methylenchlorid, Dichlorethan oder Chloroform, isomerisiert, und nach erfolgter Herstellung des jeweiligen 4-Fluorazetidinons gewünschtenfalls eventuelle Hydroxy-, Carboxy- oder Aminoschutzgruppen und/oder die Acylgruppe von $R_1$ unter Bildung eines 3-Aminoazetidinons abspaltet.

11. Verfahren zur Herstellung von p-Nitrobenzyl-3-methyl-2-(2-oxo-4-fluor-3-phenoxyacetamido-1-azetidinyl)-3-butenoat gemäß Anspruch 6, dadurch gekennzeichnet, daß man eine Lösung von p-Nitrobenzyl-3-methyl-2-(2-oxo-4-sulfino-3-phenoxyacetamido-1-azetidinyl)-3-butenoat in Methylenchlorid bei −78°C mit Perchlorylfluorid in Dimethylformamid während etwa 25 Minuten umsetzt.

12. Verfahren zur Herstellung von p-Nitrobenzyl-3-methyl-2-(2-oxo-4-fluor-3-phenoxyacetamido-1-azetidinyl)-2-butenoat gemäß Anspruch 7, dadurch gekennzeichnet, daß man das nach dem Verfahren von Anspruch 11 hergestellte p-Nitrobenzyl-3-methyl-2-(2-oxo-4-fluor-3-phenoxyacetamido-1-azetidinyl)-3-butenoat in Methylenchlorid in Gegenwart von Triethylamin durch 30 Minuten langes Rühren bei Raumtemperatur isomerisiert.

**Revendications**

1. 4-fluorazétidinone de formule 1:

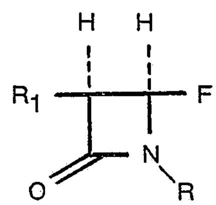

(1)

dans laquelle $R_1$ représente un groupe amino ou

1) un groupe imido de formule:

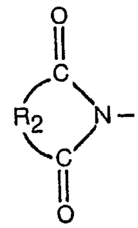

dans laquelle $R_2$ représente un groupe alkylène en $C_2$—$C_4$ ou un groupe 1,2-phénylène;

2) un groupe amido de formule:

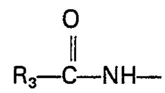

dans laquelle $R_3$ représente

a) un atome d'hydrogène, un groupe alkyle en $C_1$—$C_4$, un groupe halométhyle, un groupe cyanométhyle, un groupe benzyloxy, un groupe p-nitrobenzyloxy, un groupe t-butyloxy, un groupe 2,2,2-trichloréthoxy ou un groupe p-méthoxybenzyloxy;

b) le groupe R' qui est un groupe phényle ou un groupe phényle substitué par un ou deux atomes d'halogènes, un groupe hydroxy, un groupe hydroxy protégé, un groupe nitro, un groupe cyano, un groupe alkyle en $C_1$—$C_4$ ou un groupe alcoxy en $C_1$—$C_4$;

c) un groupe de formule:

$$R''—(Q)_m—CH_2—$$

dans laquelle R'' représente R' tel que défini ci-dessus, un groupe 1,4-cyclohexadiényle, un groupe thiényle ou un groupe furyle; m est égal à 0 ou 1 et Q représente O ou S, avec cette restriction que, lorsque m est égal à 1, R'' représente R';

d) un groupe de formule:

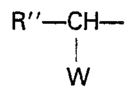

dans laquelle R'' a la signification définie ci-dessus et W représente un groupe hydroxy, un groupe hydroxy protégé, un groupe carboxy, un groupe carboxy protégé, un groupe amino ou un groupe amino protégé;

3) un groupe imidazolidinyle de formule:

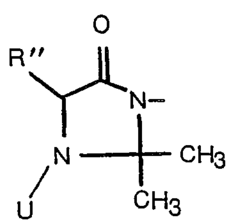

dans laquelle R'' a la signification définie ci-dessus et U représente un groupe nitroso ou un groupe acétyle; ou $R_1$ représente

4) un groupe imido de formule:

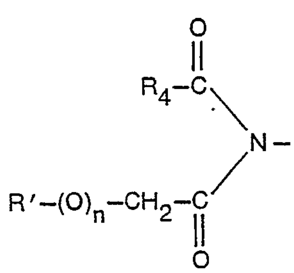

dans laquelle R′ a la signification définie ci-dessus, n est égal à 0 ou 1 et $R_4$ représente un groupe alkyle en $C_1$—$C_4$, un groupe haloalkyle en $C_1$—$C_4$, un groupe alcoxy en $C_1$—$C_4$ ou un groupe 2,2,2-trichloréthoxy; R représente un groupe butényle substitué par un groupe carboxy ou par un groupe carboxy estérifié et répondant aux formules:

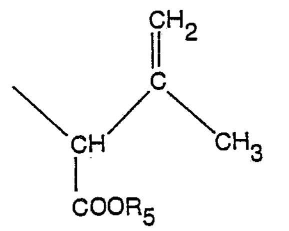

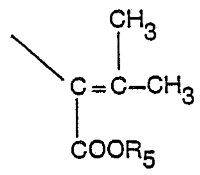

dans lesquelles $R_5$ représente un atome d'hydrogène ou un groupe protecteur du groupe carboxy; ou un sel pharmaceutiquement acceptable de cette 4-fluorazétidinone.

2. Composé selon la revendication 1, dans lequel $R_1$ est différent du groupe amino, du groupe benzyloxycarbonylamino, du groupe p-nitrobenzyloxycarbonylamino, du groupe t-butyloxycarbonylamino, du groupe 2,2,2-trichloréthoxycarbonylamino ou du groupe p-méthoxybenzyloxycarbonylamino et R représente le groupe butényle substitué par un groupe carboxy et ayant la double liaison α,β.

3. Composé selon la revendication 1, dans lequel R est un groupe de formule:

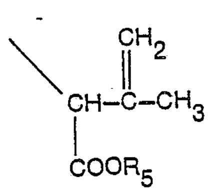

4. Composé selon la revendication 1, dans lequel R est un groupe de formule:

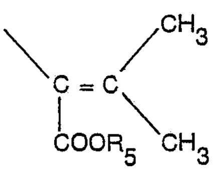

5. Composé selon l'une quelconque des revendications 1, 3 et 4 dans lequel $R_1$ est un groupe de formule:

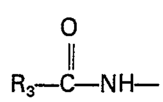

6. Dérivé de l'acide 4-fluorazétidinone-3-buténoïque de formule 1 selon la revendication 1, caractérisé en ce qu'il est choisi parmi le groupe comprenant:

le 3-méthyl-2-(2-oxo-4-fluoro-3-phénoxyacétamido-1-azétidinyl)-3-buténoate de p-nitrobenzyle,
l'acide 3-méthyl-2-(2-oxo-4-fluoro-3-phénoxyacétamido-1-azétidinyl)-3-buténoïque,
le 3-méthyl-2-(2-oxo-4-fluoro-3-phénylacétamido-1-azétidinyl)-3-buténoate de diphénylméthyle,
l'acide 3-méthyl-2-(2-oxo-4-fluoro-3-phényl-acétamido-1-azétidinyl)-3-buténoïque,
le 3-méthyl-2-(2-oxo-4-fluoro-3-p-toluamido-1-azétidinyl)-3-buténoate de diphénylméthyle,
le 3-méthyl-2-[2-oxo-4-fluoro-3-(2-thiénylacétamido)-1-azétidinyl]-3-buténoate de 2,2,2-trichloréthyle,
le 3-méthyl-2-[2-oxo-4-fluoro-3-(benzyloxycarbonylamino)-1-azétidinyl]-3-buténoate de p-méthoxybenzyle,
le 3-méthyl-2-(2-oxo-4-fluoro-3-amino-1-azétidinyl)-3-buténoate de p-méthoxybenzyle,
le 3-méthyl-2-(2-oxo-4-fluoro-3-phtalimido-1-azétidinyl)-3-buténoate de p-nitrobenzyle,
le 3-méthyl-2-(2-oxo-4-fluoro-3-acétamido-1-azétidinyl)-3-buténoate de t-butyle, et
le 3-méthyl-2-[2-oxo-4-fluoro-3-(2-t-butyloxycarbonylamino-2-phénylacétamido)-1-azétidinyl]-3-buténoate de p-nitrobenzyle.

7. Dérivé de l'acide 4-fluorazétidinone-2-buténoïque de formule 1 selon la revendication 1, caractérisé en ce qu'il est choisi parmi le groupe comprenant

le 3-méthyl-2-(2-oxo-4-fluoro-3-phénoxyacétamido-1-azétidinyl)-2-buténoate de p-nitrobenzyle,
l'acide 3-méthyl-2-(2-oxo-4-fluoro-3-phénoxyacétamido-1-azétidinyl)-2-buténoïque,
le 3-méthyl-2-[2-oxo-4-fluoro-3-(2-thiénylacétamido)-1-azétidinyl]-2-buténoate de 2,2,2-trichloréthyle,
l'acide 3-méthyl-2-[2-oxo-4-fluoro-3-(2-thiénylacétamido)-1-azétidinyl]-2-buténoïque et
l'acide 3-méthyl-2-[2-oxo-4-fluoro-3-(2-amino-2-phénylacétamido)-1-azétidinyl]-2-buténoïque.

8. Sel pharmaceutiquement acceptable d'une 4-fluorazétidinone de formule 1 selon la revendication 2.

9. Formulation antibactérienne comprenant, comme ingrédient actif, 1 à 25% en poids d'une 4-fluorazétidinone de formule 1 dans laquelle $R_1$ a la signification définie ci-dessus et est différent du groupe amino, du groupe benzyloxycarbonylamino, du groupe p-nitrobenzyloxycarbonylamino, du groupe t-butyloxycarbonylamino, du groupe 2,2,2-trichloréthoxycarbonylamino ou du groupe p-méthoxybenzyloxycarbonylamino et R est un groupe butényle substitué par un groupe carboxy et répondant à la formule:

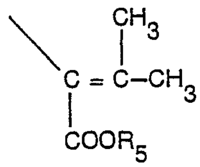

et $R_5$ est un atome d'hydrogène, ainsi qu'un ou plusieurs adjuvants ou supports pharmaceutiquement acceptables.

10. Procédé de préparation d'une 4-fluorazétidinone de formule 1 telle que décrite dans l'une quelconque des revendications 1 à 8, caractérisé en ce qu'il comprend, dans n'importe quel ordre, les étapes consistant à faire réagir, dans un solvant organique inerte aprotique et à une température se situant entre −80°C et −25°C, un composé d'azétidinone de formule 2:

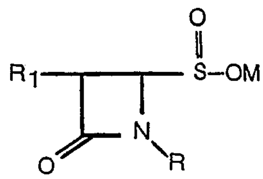

(2)

dans laquelle R représente un groupe butényle substitué par un groupe carboxy estérifié comme défini ci-dessus, $R_5$ représente un groupe protecteur du groupe carboxy, $R_1$ a la signification définie dans la revendication 1, avec cette exception qu'il ne peut être un groupe amino et que l'un ou l'autre groupe hydroxy, carboxy ou amino est présent sous forme protégée, tandis que M représente un atome d'hydrogène, un atome de sodium ou un atome de potassium, avec du fluorure de perchloryle; et, lorsqu'on le désire, si R est un 3-buténoate substitué par un groupe carboxy estérifié, isomériser en présence d'une amine tertiaire dans un solvant constitué d'un hydrocarbure inerte tel que le chlorure de méthylène, le dichloréthane ou le chloroforme; et, lorsque la 4-fluorazétidinone a été préparée, éliminer, ainsi qu'on le désire, n'importe quel groupe protecteur du groupe hydroxy, du groupe carboxy ou du groupe amino et/ou le groupe acyle de $R_1$ pour obtenir une 3-amino-azétidinone.

11. Procédé de préparation du 3-méthyl-2-(2-oxo-4-fluoro-3-phénoxyacétamido-1-azétidinyl)-3-buténoate de p-nitrobenzyle selon la revendication 6, caractérisé en ce qu'il consiste à faire réagir une solution du 3-méthyl-2-(2-oxo-4-sulfino-3-phénoxyacétamido-1-azétidinyl)-3-buténoate de p-nitrobenzyle dans du chlorure de méthylène à −78°C avec du fluorure de perchloryle dans du diméthylformamide pendant environ 25 minutes.

12. Procédé de préparation du 3-méthyl-2-(2-oxo-4-fluoro-3-phénoxyacétamido-1-azétidinyl)-2-buténoate de p-nitrobenzyle selon la revendication 7, caractérisé en ce qu'il consiste à isomériser le 3-méthyl-2-(2-oxo-4-fluoro-3-phénoxyacétamido-1-azétidinyl)-3-buténoate de p-nitrobenzyle préparé par le procédé selon la revendication 11, dans du chlorure de méthylène, en présence de triéthylamine et tout en agitant à la température ambiante pendant 30 minutes.